Europäisches Patentamt

**European Patent Office**

**Office européen des brevets**

(11) Publication number: **0 342 824**
**A1**

# EUROPEAN PATENT APPLICATION

(21) Application number: **89304520.3**

(22) Date of filing: **04.05.89**

(51) Int. Cl.4: **C12N 15/00 , C12N 1/12**

The applicant has filed a statement in accordance with Rule 28 (4) EPC (issue of a sample only to an expert). Accession number(s) of the deposit(s): ATCC - 67684 and 40452

(30) Priority: **05.05.88 US 190539**

(43) Date of publication of application:
**23.11.89 Bulletin 89/47**

(84) Designated Contracting States:
**AT BE CH DE ES FR GB GR IT LI LU NL SE**

(71) Applicant: **SCRIPPS CLINIC AND RESEARCH FOUNDATION**
**10666 North Torrey Pines Road**
**La Jolla California 92037(US)**

(72) Inventor: **Mayfield, Stephen P.**
**961 Birmingham**
**Cardiff California 92007(US)**

(74) Representative: **Fisher, Adrian John et al**
**CARPMAELS & RANSFORD 43 Bloomsbury Square**
**London WC1A 2RA(GB)**

(54) **Methods and systems for transforming chlamydomonas reinhardtii.**

(57) The present invention relates to a nucleic acid molecule that includes a codon-biased gene that is substantially free of codons having an adenine residue is the third base positions of the codons. Methods of using the nucleic acid molecule to produce C. reinhardtii transformants and C. reinhardtii transformants containing the nucleic acid molecle are also described.

FIGURE 1

## METHODS AND SYSTEMS FOR TRANSFORMING CHLAMYDOMONAS REINHARDTII

### Technical Field

The present invention relates to a nucleic acid molecule capable of transforming at high efficiencies the eukaryotic green algae, Chlamydomonas reinhardtii (C. reinhardtii). More specifically, the present invention contemplates a nucleic acid molecule that includes a selective marker gene that is essentially free of codons having adenine at the third nucleotide base positions of the codons. Methods of using the nucleic acid molecule to produce C. reinhardtii transformants and C. reinhardtii transformants containing the nucleic acid molecule are also contemplated.

### Background of the Invention

The development of a transformation system in a model photosynthetic organism would be an extremely useful tool for manufacturing polypeptides by recombinant DNA technology. Much energy has gone into developing such a transformation system in the unicellular green algae C. reinhardtii. However, to date, efforts to transform C. reinhardtii have focused on the use of bacterial or yeast selective marker genes and have been frustrated by low efficiencies and poor reproducibility.

C. reinhardtii in a simple eukaryotic organism that is an ideal model for the study of the regulation, synthesis, assembly and function of photosynthetic apparatuses. C. reinhardtii employs the same photosynthetic scheme as higher plants, contains photosynthetic complexes that are identical to those of higher plants both in function and protein components. Chug et al., Proc. Natl. Acad. Sci. USA, 72:2175-2179 (1975). However, in contrast to higher plants, C. reinhardtii has a rapid (6-24 hour) replication schedule, contains a genome that is a fraction of the size of most plant genomes, and is well characterized genetically.

Transformation is the introduction of an exogenous DNA into a cell. Transformation is usually performed in order to induce a new phenotypic trait that results from expression of a gene defined by the exogenous DNA molecule introduced into the cell. Exogenous DNA molecules capable of being introduced into a naive host cell in order to confer a new phenotype are referred to herein as "transforming DNA molecules". To aid in identifying cells into which a transforming DNA has been introduced, the transforming DNA molecule typically contains a gene whose expression results in a phenotypic trait that easily allows for separation of transformant cells from parent (non-transformed) cells. Such genes are known in the art as "selective marker genes".

Typical selective marker genes used in transforming DNA molecules are those that provide for survival selection. Survival selection can be achieved by providing resistance to a growth inhibiting substance or providing a growth factor capability to a cell deficient in such capability.

Previous attempts at transforming C. reinhardtii involved introduction into the arg 7 mutant of C. reinhardtii of a transforming DNA plasmid containing the yeast arg 4 gene as a selective marker gene. Rochaix et al., Nature, 296:70-73 (1982). The arg 4 gene of yeast encodes the enzyme argininosuccinatelyase (ASL), the last enzyme of the arginine biosynthetic pathway. The arg 7 mutant of C. reinhardtii does not have a gene capable of expressing ASL and is therefore deficient in that enzyme. Because arginine is required for growth by C. reinhardtii, the arg 7 mutant of C. reinhardtii can only be grown in a medium supplement with arginine, i.e., it is an arginine auxotroph. Successful introduction of the yeast arg 4 gene into the C. reinhardtii arg 7 mutant would therefore result in the creation of a new phenotypic trait in the mutant: the ability to survive in an arginine deficient medium, i.e., arginine prototrophy.

Rochaix et al., Supra reported that transformation of C. reinhardtii arg 7 mutants with the yeast arg 4 gene occurred with an efficiency of approximately 1 in $10^6$ treated cells, a rate similar to the spontaneous reversion rate of the arg 7 gene. Only 50% of the arginine prototrophic colonies selected contained yeast sequences by Southern blot analysis.

Rochaix et al., Cell. 36:925-931 (1984) reported transformation of the same C. reinhardtii arg 7 mutant with an autonomously replicating plasmid carrying the yeast arg 4 selective marker gene. Again, the transformation rate was low (1 in $10^6$ - $10^7$ cells). In addition, the transform DNA plasmid proved to be unstable and its presence in the cells greatly diminished after 60 generations.

Attempts at transforming C. reinhardtii with bacterial drug resistance genes have also proven futile. Reports of transformation with a cloned kanamycin resistance gene from bacteria have been published, but with equally low efficiency and with some debate on whether the introduced gene was responsible for the drug resistance or whether resistance was due to spontaneous kanamycin resistance which appears in C. reinhardtii at a high frequency. Hasnain et al., Molec. Cell. Bio., 5:3647-3650, (1985).

Although the previous reports of C. reinhardtii transformation have shown evidence for the incorporation of foreign DNA in some cases, none of these reports have shown that the introduced DNA was expressed in the transformed cells. The fact that all phenotypic traits selected in previous experiments were subject to spontaneous reversion at a frequency at or near the transformation rate suggests that these reported transformation events could have been the result of simultaneous but independent reversion of the selected trait and incorporation of the foreign DNA into the genome.

From the foregoing it can be seen that the art has not been able to determine the reason(s) for the inability to efficiently produce stable C. reinhardtii transformants. It is not clear whether the problem(s) lie in the introduction, integration or expression stages of the transformation process.

A gene of particular interest to the present invention is the C. reinhardtii psb1 gene which encodes oxygen evolving enhancer protein number one (OEE1): Mayfield et al., EMBO, 6:313-318 (1987). OEE1 is part of the photosystem II reaction center core and has recently been shown to be required for photosynthetic oxygen evolution. C. reinhardtii cells deficient in OEE1 are incapable of photoautrotrophic growth, i.e., the OEE1 protein is absolutely required for photosynthesis.

The C. reinhardtii nuclear mutant FuD44 lacks the ability to express OEE1 and is therefore completely deficient in photosynthetic oxygen evolution. Kuwabara et al., Biochem. Biophys. Acta., 581:228-215 (1979). In FuD44, a 5 kilobase (kb) DNA insertion into the 5′ region of the psbI gene results in the complete absence of OEE1 mRNA and protein. Mayfield et al., Supra.

## Summary of the Invention

The present invention contemplates a nucleic acid molecule suitable for transforming C. reinhardtii comprising a gene , preferably a selective marker gene, that is substantially free of codons having an adenine residue in the third base positions of the codons.

A nucleic acid molecule suitable for transforming C. reinhardtii consisting essentially of no more than about 8,500 base pairs and containing a gene having an exon and an intron, the exon being substantially free of codons having an adenine residue in the third base positions of the codons is also contemplated.

Further contemplated is a nucleic acid molecule comprising a vector operatively linked to a first structural gene and to a second structural gene wherein the structural genes are substantially free of codons having an adenine residue in the third base positions of the codons.

In another embodiment, the present invention contemplates a recombinant nucleic acid molecule comprising a vector operatively linked to a transforming nucleic acid molecule, the transforming nucleic acid molecule consisting essentially of no more than about 8,500 base pairs and containing a gene that is substantially free of codons having an adenine residue in the third base positions of the codons.

Also contemplated is a nucleic acid molecule suitable for transforming C. reinhardtii comprising a heterologous gene that is substantially free of codons having an adenine in the third base positions of the codons.

The present invention also contemplates a method of producing a cloned C. reinhardtii transformant comprising:

(a) introducing a nucleic acid molecule or recombinant nucleic acid molecule of this invention into at least one member of a population of C. reinhardtii cells and producing a transformant-containing population;

(b) cloning and retaining a cell of the transformant-containing population that displays the selectable phenotype induced by the nucleic acid molecule.

Still further contemplated is a C. reinhardtii cell transformed with a nucleic acid molecule or recombinant nucleic acid molecule of this invention.

## Brief Summary of the Drawings

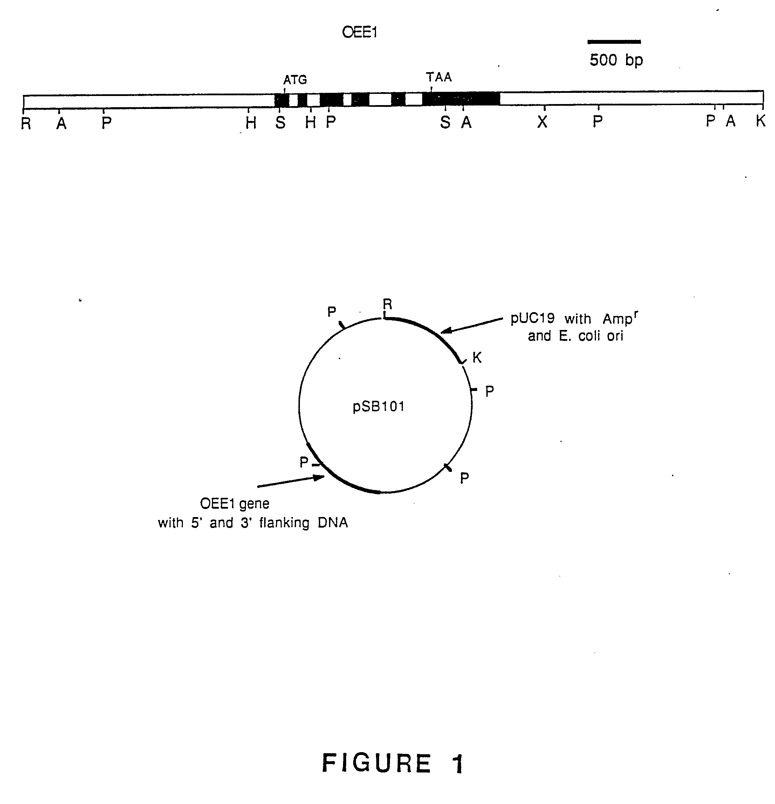

Figure 1 is a schematic representation of an 8.2 kilobase (kb) transforming nucliec acid molecule containing the cloned OEE1 gene. The lower portion of the figure illustrates the recombinant DNA plasmid pSB101 that inclludes the 8.2 kb OEE1 gene-containing transforming nucleic acid molecule.

The top portion of the figure illustrates the location of introns (open boxes) and exons (closed boxes) within the OEE1 structural gene. The approximate locations of the amino acid residue sequence start (ATG) codon and stop (TAA) signal are also shown. The approximate location of restriction endonuclease sites within the 8.2 kb fragment and pSB101 are indicated as follows: Eco RI (R), Ava I (A), Pst I (P), Hind III (H), Sal I (S), Xho I (X) and Kpn I (K). An approximately 500 nucleotide base pair (bp) bar is also shown for scale.

The bottom portion of the figure illustrates the location of the 8.2 kb EcoR I-Kpn I fragment and pUC$_{19}$ plasmid clong vector sequences within the pSB101 recombinant plasmid. The location of the pUC19-derived ampicillinase selectable marker (Amp$^r$) gene and a plasmid origin of replication (E. coli ori) are also shown.

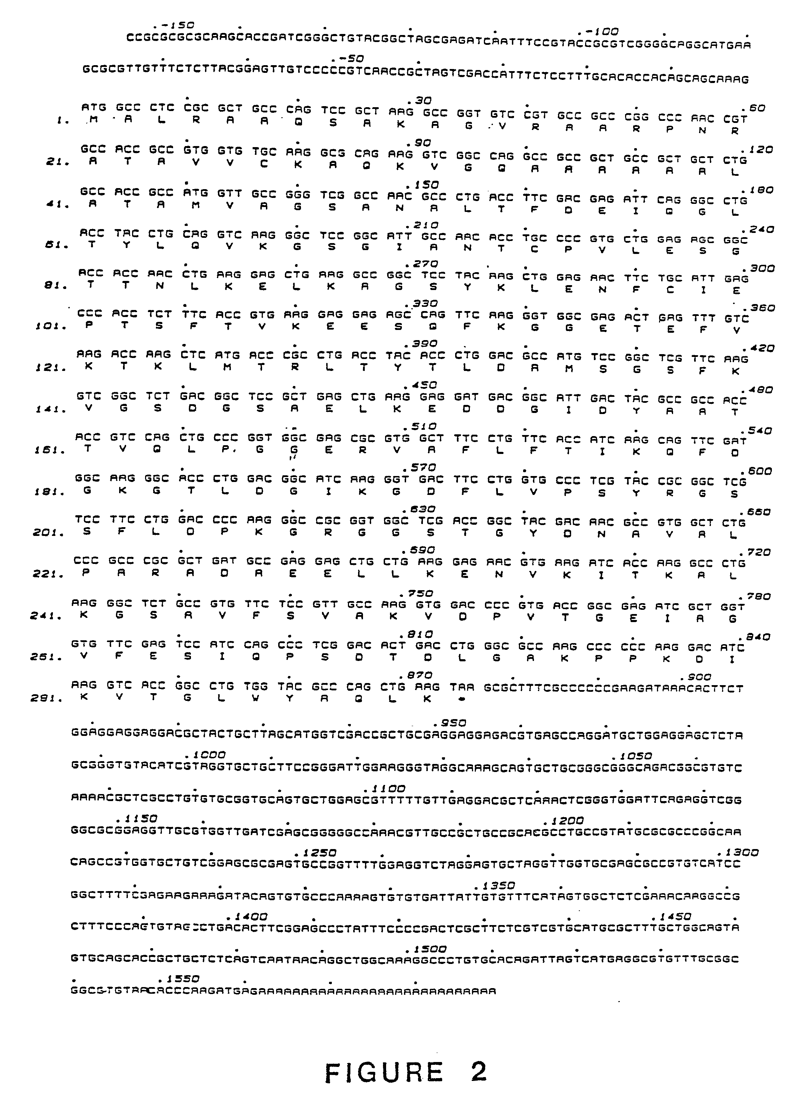

Figure 2 illustrates the nucleotide sequence of a cDNA that codes for the OEE1 protein and the deduced amino acid residue sequence of the protein. The nucleotide sequence is shown from left to right and in the direction of 5′ terminus to 3′ terminus using the single letter nucleotide base code represented as an uninterrupted linear series of bases from base -153 to base 1589, the base numbers being shown above the linear sequence. The deduced amino acid residue sequence of the OEE1 protein is shown from left to right and in the direction from amino-terminus to carboxy-terminus using the single letter amino acid residue code represented as an uninterrupted linear series of residues. from residue 1 (M) at the amino-terminus to residue 292 (K) at the carboxy-terminus, the residue numbers being shown in the margin to the left of the sequence.

The reading frame is indicated by the placement of the deduced amino acid residue sequence below the nucleotide sequence such that the single letter that represents each amino acid residue is locate below the second (middle) base in the corresponding codon.

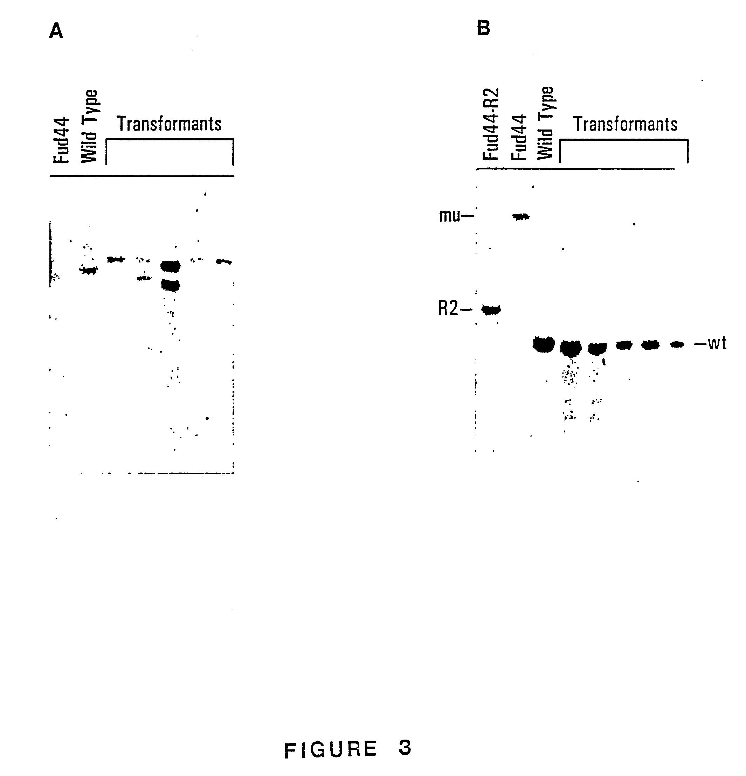

Figure 3 illustrates an autoflourogram of a Southern blot analysis of C. reinhardtii transformants as described in Example 2C. Genomic DNA from wild type (wt), mutant FuD44 (mu), a spontaneous revertant of FuD44 (FuD44-R2; R2), and 5 transformants chosen at random were restriction endonuclease digested using Eco RI plus Kpn I (panel A) or Pst I (panel B), electrophoresed on agarose gels, blotted onto nitrocellulose and hybridized with a [$^{32}$P]-labeled OEE1-specific probe.

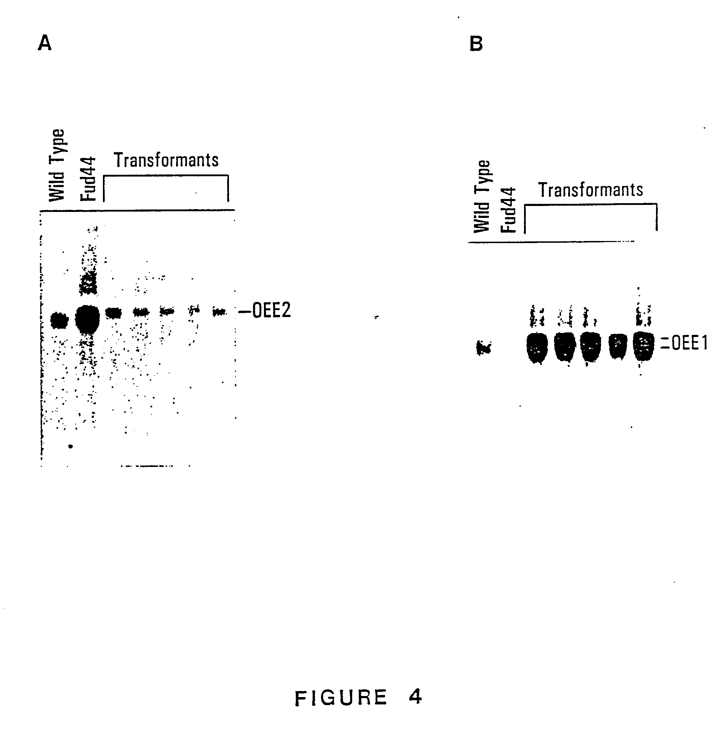

Figure 4 illustrates an autoflourogram of a Northern blot analysis of C. reinhardtii transformants as described in Example 2C. RNA was isolated from wild type, mutant FuD44 and 5 transformants chosen at random, electrophoresed on denaturing formaldehyde gels, electroblotted onto nylon membranes and hybridized using a [$^{32}$P]-labelled OEE2-specific (panel A) or OEE1-specific (panel B) probe.

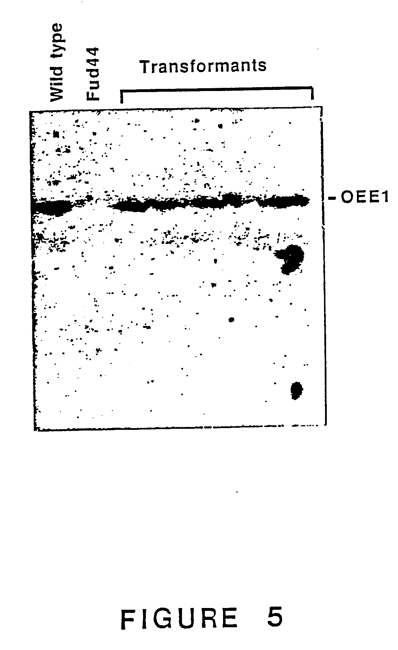

Figure 5 illustrates an immunoblotting analysis of OEE1 proteins expressed in wild type, mutant FuD44 and 5 transformants selected at random, wherein said proteins are electrophoresed on polyacrylamide gels in the presence of SDS (SDS-PAGE), electroblotted onto nitrocellulose and visualized by OEE1-specific antibodies as described in Example 2C.

Detailed Description of the Invention

A. Definitions

Gene: Refers to a nucleic acid sequence capable of expressing, in a compatible host, a polypeptide. Thus, a gene contains an expression control region operatively linked to a structural gene.

Structural Gene: Refers to that portion of a gene containing the DNA sequence that encodes the amino acid residue sequence of the polypeptide expressed by the gene. A structural gene can contain one or more introns.

Intron: Is a portion of a structural gene that is transcribed but does not appear in the final (translated) mRNA transcript, and thus contains no codons.

Exon: Is a portion of a gene that codes for the translated mRNA transcript and thus contains codons.

Codon: A group of three adjacent nucleotide residues that codes for an amino acid residue in the polypeptide expressed by a gene. Within a codon, the 5′, middle and 3′ nucleotide residue positions are numbered one, two and three, respectively, and are typically referred to herein as the first, second and third base positions of a codon, respectively.

Nucleotide: A monomeric unit of DNA or RNA consisting of a sugar moiety (pentose), a phosphate, and a nitrogenous heterocyclic base. The base is linked to the sugar moiety via the glycosidic carbon (1′ carbon of the pentose) and that combination of base and sugar is a nucleoside. When the nucleoside contains a phosphate group bonded to the 3′ or 5′ position of the pentose it is referred to as a nucleotide.

Base Pair (bp): A partnership of adenine (A) with thymine (T), or of cytosine (C) with guanine (G) in a double stranded DNA molecule. In RNA molecules, uracil (U) replaces thymine.

## B. Nucleic Acid Molecules

In one embodiment, a nucleic acid molecule of the present invention is suitable for transforming C. reinhardtii. A transforming nucleic acid molecule is further characterized as containing a gene that is substantially free of codons having an adenine residue in the third base positions of the codons, i.e., a codon-biased gene.

By substantially free is meant that the structural gene is at least about 95%, preferably at least about 99% and more preferably at least about 99.9% free of codons in the third base position. The codon-biased gene can be either homologous (found in wild type C. reinhardtii) or heterologous (not found in wild type C. reinhardtii) to C. reinhardtii. Preferably, the codon biased gene is a selective marker gene, and more preferably is a survival selection marker gene.

Preferred survival selection marker genes include the OEE1 gene (i.e., psbl), the acetolactate synthase (ALS) gene, the argininosuccinatelyase (ASL) gene, the chloramphenicol acetyltransferase (CAT) gene, and the like. In preferred embodiments, the codon-biased gene contains a predetermined endonuclease restriction site into which a nucleic acid molecule can be ligated to disrupt expression of the codon-biased gene.

In preferred embodiments, a transforming nucleic acid molecule consists substantially of no more than about 7,500 base pairs (bp), preferably no more than about 8,000 bp, and more preferably no more than about 8,500 bp.

Further preferred is a transforming nucleic acid molecule containing a codon-biased gene having an exon and an intron, with the exon(s) being essentially free of codons having an adenine residue in the third base positions of the codons.

Still further preferred is a transforming nucleic acid molecule wherein the amino acid residue sequence-coding portion of the codon-biased gene consists essentially of a plurality of codons, each of which contains adenine, thymidine, guanosine or cytosine in the first and second base position within each codon, and thymidine, guanosine or cytosine at the third base position within each codon.

In the most preferred embodiments, the codons of the transforming nucleic acid molecule's codon-biased gene are all members of the group consisting of:
GCT, GCC, CGC, AAC, GAC, TGC, CAG, GAG, GGC,
CAC, ATT, ATC, CTG, AAG, ATG, TTC, CCC, TCC,
TCG, ACC, TGG, TAC, GTC, and GTG.

A preferred transforming nucleic acid molecule consists essentially of the OEE1 gene-containing 8.2 kilobase (kb) EcoR I-Kpn I restriction fragment of plasmid pSB101. Eschericia coli transformed with plasmid pSB101 have been deposited with the American Type Culture Collection (ATCC), Rockville, MD on May 3, 1988 and assigned the accession number 67684.

A transforming nucleic acid molecule of this invention can be prepared using methods well known in the art. For instance, a nucleic acid molecule containing a desired gene can be cloned from the nuclear genome of wild type C. reinhardtii.

In addition, a chemical synthesis technique, such as the phosphotriester method of Matteucci et al., J. Am. Chem. Soc., 103:3185 (1981) can be used to prepare a gene or structural gene that encodes a protein of known amino acid residue seuqence. Chemical synthesis techniques are particularly well adapted to forming genes substantially free of codons having adenine in the third base positions of the codons because they permit formation of nucleic acid molecules having predetermined sequences.

## C. Recombinant Nucleic Acid Molecules

A recombinant nucleic acid molecule of the present, invention comprises a vector operatively linked to a first structural gene, and preferably also operatively linked to a second structural gene. The first operatively linked structural gene, and second structural gene if present are substantially free of codons having an adenine residue in the third base positions of the codons (codon-biased structural genes). Preferably, at least one of the operatively linked structural genes is a selective marker gene, and more preferably is a survival selection marker gene. The operatively linked structural gene(s) can be either homologous or heterologous to C. reinhardtii.

In preferred embodiments a codon-biased structural gene contains a predetermined endonuclease restriction site into which a nucleic acid molecule can be ligated to disrupt expression of the codon-biased structural gene. Of course, in embodiments where an entire gene is operatively linked to a vector, the predetermined restriction site can be in the promoter region of the linked gene.

As used herein, the term "vector" refers to a nucleotide molecule having a regulatory element that directs replication of the vector genome and to which another nucleotide molecule can be operatively linked so as to bring about replication of the linked molecule. Vectors and their use in cell transformation are well known in the art, and include DNA plasmid vectors, bacteriophage vectors, retroviral vectors, and the like. Typically, vectors range in size from about I kilobase (kb) to about 200 kb. Vectors capable of directing or permitting expression of an operatively linked structural gene are referred to herein as "expression vectors".

The choice of vector to which a nucleic acid molecule containing a codon-biased structural gene is operatively linked depends directly, as is well known in the art, on the functional properties desired, e.g., replication and/or protein expression, and the host cell to be transformed, these being limitations inherent in the art of constructing recombinant nucleic acid molecules. However, a vector contemplated by the present invention is at least capable of directing the replication, and preferably also expression, of a codon-biased structural gene included in nucleic acid segment to which the vector is operatively linked.

In preferred embodiments, a vector contemplated by the present invention includes an origin of replication (replicon), i.e., a DNA sequence having the ability to direct autonomous replication and maintenance of the recombinant nucleic acid molecule extrachromosomally in a host cell, such as a bacterial or C. reinhardtii host cell, transformed therewith. Such replicons are well known in the art. In addition, those embodiments that include a procaryotic replicon typically also include a gene whose expression confers drug resistance to a bacterial host transformed therewith. Typical bacterial drug resistance genes are those that confer resistance to ampicillin or tetracycline.

Those vectors that include a procaryotic replicon can also include a procaryotic promoter capable of directing the expression (transcription and translation) of a codon-biased gene in a bacterial host cell, such as E. coli, transformed therewith. A promoter is an expression control element formed by a DNA sequence that permits binding of RNA polymerase and transcription to occur. Promoter sequences compatible with bacterial hosts are typically provided in plasmid vectors containing convenient restriction sites for insertion of a DNA segment of the present invention. Typical of such vector plasmids are pUC8, pUC9, pBR322 and pBR329 available from Biorad Laboratories, (Richmond, CA).

The construction and use of retroviral vectors has been described by Sorge et al., Mol. Cell. Biol., 4:1730-37 (1984).

A variety of methods have been developed to operatively link nucleic acid molecules to vectors via complementary cohesive termini. For instance, complementary homopolymer tracts can be added to the nucleic acid segment to be inserted and to the vector nucleic acid. The vector and nucleic acid segment are then joined by hydrogen bonding between the complementary homopolymeric tails to form recombinant nucleic acid molecule.

Synthetic linkers containing one or more restriction sites provide an alternative method of joining a DNA segment to DNA vector. The DNA segment, generated by endonuclease restriction digestion, is treated with bacteriophage T4 DNA polymerase or E. coli DNA polymerase I, enzymes that remove protruding, $3'$, single-stranded termini with their $3'-5'$ exonucleolytic activities and fill in recessed $3'$ ends with their polymerizing activities. The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies, Inc., New Haven, CN.

## D. Transformed Cells and Cultures

The present invention also relates to a host cell transformed with a transforming nucleic acid molecule or a recombinant nucleic acid molecule of the present invention. (For ease of expression, the transforming nucleic acid molecules and recombinant nucleic acid molecules of this invention are sometimes referred to herein using the phrase "subject nucleic acids" in its various grammatical forms.) The host cell can be either procaryotic or eucaryotic. Bacterial cells are preferred procaryotic host cells and typically are a strain of E. coli such as, for example the E. coli strain DH5 available from Bethesda Research Laboratories, Inc., Bethesda, MD. Preferred eucaryotic host cells include C. reinhardtii cells. Transformation of appropriate cell hosts with a subject nucleic acid is accomplished by well known methods that typically depend on the cell host and type of vector used. With regard to transformation of procaryotic host cells, see, for example, Cohen et al., Proc. Natl. Acad. Sci. USA, 69:2110 (1972); and Maniatis et al., Molecular Cloning, A Laboratory Mammal, Cold Spring Harbor Laboratory, Cold Spring Harbor, NY (1982). With regard to transformation of cells with retroviral vectors, see, for example, Sorge et al., Mol. Cell. Biol., 4:1730-37 (1984); Graham et al., Virol., 52:456 (1973); and Wigler et al., Proc. Natl. Acad. Sci. USA, 76:1373-76 (1979). .

Successfully transformed cells, i.e., transformants, can be identified by well known techniques. For example, cells resulting from the introduction of a subject nucleic acid can be cloned to produce monoclonal colonies. Cells from those colonies can be harvested, lysed and their DNA content examined for the presence of the subject nucleic acid (or its DNA equivalent) using a method such as that described by Southern, J. Mol. Biol., 98:503 (1975) or Berent et al., Biotech., 3:208 (1985).

In addition to directly assaying for the presence of the subject nucleic acid, successful transformation can be confirmed by well known immunological methods when the subject nucleic acid is capable of directing the expression a structural gene it contains. For example, cells successfully transformed with an expression vector produce an antigenic protein product. Samples of cells suspected of being transformed are harvested and assayed for the structural gene antigenic protein product using antibodies specific for that antigen.

Thus, in addition to the transformed host cells themselves, the present invention also contemplates a culture of those cells, preferably a monoclonal (clonally homogeneous) culture, or a culture derived from a monoclonal culture, in a nutrient medium. Preferably, the culture also contains a recombinantly produced polypeptide product.

Nutrient media useful for culturing transformed host cells are well known in the art and can be obtained from several commercial sources.

Methods for recovering an expressed protein from a culture are well known in the art and include fractionation of the protein-containing portion of the culture using well known biochemical techniques.

For instance, the methods of gel filtration, gel chromatography, ultrafiltration, electrophoresis, ion exchange, affinity chromatography and the like, such as are known for protein fractionations, can be used to isolate the expressed proteins found in the culture. In addition, immunochemical methods, such as immunoaffinity, immunoadsorption and the like can be performed using well known methods.

E. Methods for Producing C. reinhardtii Transformants

The present invention contemplates a method for producing a cloned C. reinhardtii transformant. The method comprises introducing a subject nucleic acid into at least one member of a population of C. reinhardtii cells, thereby producing a transformant containing population.

Methods for introducing a subject nucleic acid into C. reinhardtii include those typically used for bacterial and vertebrate cells. Briefly, those methods typically involve admixing the subject nucleic acid, preferably the 8.2 kb EcoR I-Kpn I fragment of plasmid pSB101, to be introduced with naive C. reinhardtii cells preferably FuD44 cells. The admixture is treated with a transformation-facilitating agent, such as calcium chloride, an electric field, and the like, for a time period sufficient for the subject nucleic acid to enter at least one member of the population. See, for example, the electroporation method described in Miller et al., Proc. Natl. Acad Sci USA 85:856-860 (1988).

In preferred embodiments, the transformant-containing population is treated, within about one hour, preferably within about 30 minutes and more preferably within about 15 minutes of the transforming nucleic acid being introduced into the cell, with a mutagenic agent such as ultraviolet radiation, x-ray radiation hydroxylamine, nitroguanidine, bromouracil, nitrous acid and the like.

A cell of the transformant-containing population that displays the phenotype induced by the subject nucleic acid introduced is then cloned, typically by limiting dilution, and retained. Where the introduced subject nucleic acid contains a survival selection marker gene capable of being expressed in a C. reinhardtii transformant, cloning is usually accomplished by first maintaining (culturing) the transformant-containing population in a medium that will not support non-transformed cells for a time period sufficient for any non-transformed cells to die. The surviving cells are selected, cloned and a clone subsequently retained.

F. Methods for Producing Recombinant Polypeptides

Another aspect of the present invention pertains to a method for producing polypeptides, preferably polypeptides heterologous to C. reinhardtii using C. reinhardtii transformants.

The method entails initiating a culture comprising a nutrient medium containing a C. reinhardtii heterologous protein expressing transformant of the present invention. The culture is maintained for a time period sufficient for the transformants to express a polypeptide coded for by the subject nucleic acid used to produce the transformant. The expressed protein is then recovered from the culture.

## G. Expression Promoters

In another embodiment, the present invention contemplates a promoter DNA molecule of about 3000 base pairs comprising the $E_coR$ I-Sal I OEE1-derived endonuclease restriction fragment of plasmid pSB101. By "OEE1-derived" is meant that the promoter DNA molecule contains the DNA sequence shown in Figure 2 from nucleotide residue position -153 to nucleotide residue position -37 at its 3'-terminus.

Further contemplated is an expression promoting nucleic acid molecule suitable for transforming C. reinhardtii comprising a promoter DNA molecule operatively linked to a structural gene heterologous to the promoter and EcoR I-Sal I OEE-derived endonuclease restriction fragment of plasmid pSB101. By "structural gene heterologous to the promoter" is meant a gene other than the OEE1 structural gene.

Also contemplated are methods for transforming C. reinhardtii and C. reinhardtii transformants that utilize or contain, respectively an expression promoting nucleic acid molecule of this invention, as well as an expression vector comprising a promoter DNA molecule of this invention operatively linked to a replicon.

## Examples

The following examples are intended to illustrate, but not limit, the present invention.

## 1. Construction of Plasmid pSB101 Containing the Gene That Encodes The OEE1 Protein

Recombinant plasmid pSB101 was constructed using methods that. are well known in the art. See, for example, Maniztis et al., Molecular Cloning: A Laboratory Manual, Cold Spring Harbor Laboratories, Cold Spring Harbor, NY (1983).

Using the wild type C. reinhardtii strain 137c, obtained from the Chlamydomonas Stock Center (Botany Dept, Duke University, Durhan, NC), an EMBL3 genomic library containing the wild type OEE1 gene was constructed as described by Mayfield et al., EMBO J., 6:313-318 (1987). The EMBL3 vector linked to the OEE1 gene was restriction endonuclease digested to completion with EcoR I and Kpn I to yield the 8.2 kilobase (kb) DNA segment (EcoR I-Kpn I restriction fragment) illustrated in Figure 1.

The 8.2 kb EcoR I-Kpn I DNA segment was ligated into (operatively linked to) the plasmid vector, pUC19 (Pharmacia, Piscataway, NJ), at pUC19's EcoR I and Kpn I restriction sites, to form recombinant plasmid pSB101, a preferred recombinant nucleic acid molecule of the present invention, whose structure is schematically diagrammed in Figure 1. pSB101 was introduced into E.coli strain JM109 (Pharmacia) by routine bacterial transformation procedures.

## 2. Transformation of C. reinhardtii Mutant, FuD44. Using The Selectable Marker, Gene OEE1

## à. Preparation of Autolysin

Autolysin was prepared by the following means using two separate cultures, mating type ( + ) and mating type (-), of wild type C. reinhardtii. Wild type C. reinhardtii strain 137c mating types ( + ) and (-) (also referred to as wt$^+$ or wt$^-$) were obtained as separate cultures from the Chlamydomonas Stock Center (Duke University, Durham, NC) and cultured in TAP medium.

Concentrated Beijerinck solution (4XBeijerinck) was prepared by admixing 16 gm $NH_4Cl$, 2 gm $CaCl_2 \cdot 2H_2O$ and 2 gm $MgSO_4 \cdot 7H_2O$ into 1 liter of water.

Concentrated potassium phosphate solution (2XPO$_4$) was prepared by admixing 14.34 gm $K_2HPO_4$, 7.26 gm $KH_2PO_4$ and sufficient concentrated KOH to adjust the pH to 6.9 into 1 liter of water.

Trace elements solution was prepared by (a) admixing in 550 milliliters (ml) water, in the following order, 11.4 gm of $H_3BO_3$, 22 gm of $ZnSO_4 \cdot 7H_2O$, 5.06 gm of $MnCl_2 \cdot 4H_2O$, 4.99 gm of $FeSO_4 \cdot 7H_2O$, 1.61 gm of $CoCl_2 \cdot 6H_2O$, 1.57 gm of $CuSO_4 \cdot 5H_2O$ and 1.1 gm of $(NH_4)_6 M_{o7}O_{24} \cdot 4H_2O$, and then heating the admixture to 100 degrees C, (b) dissolving 50 gm EDTA [ethylenediaminetetraacetic acid (disodium)] in 250 mls water by admixture and heating and then admixing the heated solution to solution (a) to produce solution ab, (c) cooling the solution ab to 80 degrees C and adjusting its pH to 6.5 - 6.8 using 20% KOH, (d) adjusting the final volume of solution ab to 1 liter and then maintaining the volume adjusted solution at room temperature for 2 weeks, and (e) filtering the maintained solution 3 times over 3 layers of whatman #1 filter paper (Whatman, Clifton, NJ) under suction.

TAP medium was prepared by admixing 975 mls water, 2.42 gms Tris-HCl, 25 mls 4XBeijerinck solution, 1 ml IM potassium phosphate (pH 7.0), 1 ml trace elements solution and 1 ml glacial acetic acid.

Six 90 millimeter (mm) disposable petri dishes (Fischer Scientific, Pittsburg, PA) containing TAP medium in an agar support (20% w/v Agar; DIFCO, Detroit, MI) were innoculated with $4 \times 10^6$ wt[+] cells, and a parallel set of dishes similarly innoculated with wt[-] cells. Both sets were grown for 4 days under high light (HL; flourescent lights of about 2000-4000 lux). Thereafter the cells on each plate were resuspended using 20 ml of NFHSM1/2PO$_4$ medium and each mating type pooled. The mating type pools were then cultured by continuous agitation using a rotary shaker (150 rpm) under HLL.

NFHSM1/2PO$_4$ medium was prepared by admixing 974 ml water, 25 ml 2XPO$_4$ and 1 ml trace elements solution.

The wt[+] and wt[-] cells were cultured until at least about 80% of the cells of each culture were capable of mating behavior. Mating behavior capability was measured by admixing a small aliquot of wt[+] cells with wt[-] cells and visually observing under a light microscope the proportion of the total cells that engage in mating behavior, i.e., exhibit pairwise coupling as opposed to maintaining a unicellular and motile status (non-coupling). After 2 hours of rotary agitation, the pooled cultures of wt[+] and wt[-] cells were each separately sampled, the cell concentration was determined for each, and each culture was centrifuged at 2000 rpm for 10 minutes at room temperature using a Beckman 13.1 rotor (Beckman Instruments, Palo Alto, CA). The resulting pellets were resuspended in NFHSM1/2PO$_4$ medium at $1 \times 10^8$ cells/ml, and the rotary agitation continued under HL until both cultures matured, i.e., until between 80 and 90% of the cells displayed mating behavior capability.

Equal amount of the mature wt[+] and wt[-] cultures were admixed and maintained without agitation for 4 minutes. Thereafter the admixture was centrifuged for 5 minutes at 4 degrees C in a Beckman 13.1 rotor at 2000 rpm to separate the cells from the culture supernatant. The supernatant was collected and recentrifuged at 17,000 rpm for 15 minutes at 4 degrees C using a SS-34 Sorvall rotor (DuPont, Wilmington, DE), and the resulting supernatant was collected to form autolysin solution.

### b. Transformation of FuD44 Using Plasmid pSB101

Mutant C. reinhardtii FuD44 was prepared from wild type 137c by 5-flourodeoxyuridine and metronidazole treatment and was cultured in TAP mediua as described by Mayfield et al., EMBO J., 6:313-318 (1987). Cultures of FuD44 were deposited with the ATCC on May 3, 1988, and were given the accession number 40452.

Cultures of FuD44 were grown to approximately $IX10^7$ cells per ml in TAP media, and 20 mls of this culture was centrifuged at 2000 rpm for 5 minutes in a 13.1 rotor to pellet the cells. The cell pellet was resuspended in 4.0 mls of autolysin solution, and the resuspended cells were maintained on a rotary shaker for 1 hour at room temperature to allow sufficient digestion of the FuD44 cell walls to form protoplast containing-solutions.

Protoplast containing-solutions were then diluted by admixture with TAP medium to a concentration of $10^7$ cells per ml to form a solution that also contained, by further admixture, the following: (1) carrier DNA prepared from FuD44 and added at a concentration of 25 ug/ml, (2) 7.5% PEG 6000 (polyethylene 6000, autoclaved; Sigma Chemical Co, St. Louis, MO), and (3) pSB101 DNA digested to completion with Eco RI and KpNI and added to a concentration of 10 ug/ml. One ml of the diluted cells were placed on ice in disposable cuvettes and subjected to a single electrical pulse across a 4 millimeter (mm) gap from a 500 microfarad (uF) capacitor charged to about 200 volts using a transfector 300 electroporation system (BTX, San Diego, CA).

After electroporation, cell cultures were maintained for 1 hour on ice, and then 3 mls of TAP media was added to the cells and the cultures were maintained for 16-24 hours under HL with rotary agitation at room temperature. The cultures were then pelleted at 2000 rpm as before, the cell pellet was resuspended in 250 ul HSM media (prepared by admixing 725 mls water, 25 ml 4X Beijerinck solution, 50 mls 2XPO$_4$ and 1 ml

trace elements solution) and the resuspended cells plated onto a 90 mm petri dish containing HSM media in 20% agar. Plated cells were maintained at room temperature under HL for 10 to 14 days after which time transformant FuD44 cells grew and formed visible discreet colonies, hereinafter called transformants.

The above transformation procedure typically yielded about 50 to 150 transformant colonies per plate with a maximum of about 175 transformants.

A significant increase in transformation efficiency was observed when the culture was exposed to ultraviolet (UV) light about 24 hours after electroporation. This modified procedure comprised adding a 10 minute wait after plating the electroporated FuD44 cells onto HSM plates, followed by a 10 minute exposure to direct (dishes uncovered) UV light emitted from a 48 inch, 30 watt germicidal lamp (model 630T8, General Electric, Cleveland, OH) at a distance of about three feet. After the UV exposure, the dishes were recovered and maintained under HL at room temperature for 10-14 days as described above.

Using this modified procedure, approximately 250-300 transofrmants were observed per plate and in a few cases there were as many as 500 transformants per plate.

c. Verification of Transformation of FuD44 Cells by The OEE1 Selectable Marker Gene

Five transformants, produced as described in Example 2b, were randomly chosen for further characterization to demonstrate that the OEE1 selectable marker gene was stably introduced into the naive FuD44 cells to produce transformants capable of expressing the OEE1 gene product.

Cellular DNA was isolated from wild type C. reinhardtii. mutant FuD44, mutant FuD44-R2 and each of the five transformants using well known techniques. Eath isolaated DNA was subjected to restriction endonuclease digestion and analyzed by Southern blotting using a OEE1 gene-specific probe as described in the legend to Figure 3. The probe used hybridizes to a 5 kb Pst I FuD44 cellular DNA fragment restriction and a 0.5 kb Pst I FuD44-R2 cellular DNA restriction fragment.

Southern analysis of the isolated DNA's digested with EcoR I and Kpn I indicated that there were no transformants having a pattern that appeared exactly like the wild type restriction fragment pattern. (Figure 3, panel A).

As shown in panel B of Figure 3, the cellular DNA of all of the transformants contain the same size Pst I restriction fragment as the wild type. However, Figure 3 also demonstrates that both mutants contain larger Pst I fragments indicating the presence of a gene-disrupting insert within the wild type OEE1 gene.

Messenger RNA (mRNA) was isolated from each of the five selected transformants and analyzed by Northern blot hybridization as described in the legend to Figure 4. Probing the Northern blot with an OEE2-specific probe demonstrated that FuD44 cells, and the transformants, appear to express the same OEE2 mRNA as wild type (Figure 4, panel). The Northern blots results shown in Figure 4, panel B indicate that all of the transformants accumulated an OEE1 specific messenger RNA that was indistinguishable from wild type, in contrast to the mRNA species expressed in non-transformed mutants.

These data indicate the presence of OEE1 gene sequences in the transformants. Furthermore, the levels of OEE1 mRNA in the transformants appears significantly greater than in wild type cells demonstrating the potency of expression when utilizing this system for selective marker expression.

To analyze protein expression in transformed cells, immunoblots were prepared of cell lysates that had been subjected to sodium dodecyl sulfate-polyacrylamide gel electrophoresis (SDS-PAGE). The preparation of cell lysates, SDS-PAGE conditions, protein blotting and antibody immunoblotting procedures used herein are described in Mayfield et al., EMBO J. 6:313-318 (1987).

The results of the immunoblot study, shown in Figure 5, indicate that the OEE1 protein was expressed in all the transformed cell lines and is immunologically indistinguishable from the wild type OEE1 protein. As expected, no OEE1 protein was expressed in non-transformed FuD44 cells.

The foregoing specification, including the specific embodiments and examples, is intended to be illustrative of the preent invention and is not to be taken as limiting. Numerous other variations and modifications can be effected without departing from the true spirit and scope of the present invention.

**Claims**

1. A nucleic acid molecule suitable for transforming Chlamydomonas reinhardtii comprising a gene that is substantially free of codons having an adenine residue in the third base positions of said codons.

2. The nucleic acid molecule of claim 1 wherein all codons of said gene are members of the group consisting of:

GCT, GCC, CGC, AAC, GAC, TGC, CAG, GAG, GGC,
CAC, ATT, ATC, CTG, AAG, ATG, TTC, CCC, TCC, TCG, ACC,
TGG, TAC, GTC, and GTG.

3. The nucleic acid molecule of claim 2 wherein said gene is the OEE1 gene of Chlamydomonas reinhardtii.

4. A nucleic acid molecule suitable for transforming Chlamydomonas reinhardtii consisting essentially of no more than about 8,500 base pairs and containing a gene having an exon and an intron, said exon being substantially free of codons having an adenine residue in the third base positions.

5. A nucleic acid molecule consisting essentially of the EcoR I-Kpn I restriction fragment of plasmid pSB101.

6. A recombinant nucleic acid molecule comprising a vector operatively linked to a structural gene and to a selective marker gene wherein said structural gene and said selective marker gene are substantially free of codons having an adenine residue in the third base positions of said codons.

7. A recombinant nucleic acid molecule comprising a vector operatively linked to a transforming DNA molecule, said transforming DNA molecule consisting essentially of no more than about 8,500 base pairs and containing a gene that is substantially free of codons having an adenine residue in the third base positions of said codons.

8. The nucleic acid molecule of claim 7 that is the plasmid pSB101.

9. A nucleic acid molecule suitable for transforming Chlamydomonas reinhardtii comprising a gene that is heterologous to Chlamydomonas reinhardtii. said gene being substantially free of codons having an adenine in the third base positions of said codons.

10. The nucleic acid molecule of claim 9 wherein said heterologous gene is a survival selection marker gene.

11. A recombinant nucleic acid molecule comprising a vector operatively linked to a structural gene that is heterologous to Chlamydomonas reinhardtii said structural gene being substantially free of codons having an adenine in the third base positions of said codons.

12. A method of producing a cloned Chlamydomonas reinhardtii transformant comprising:

(a) introducing a nucleic acid molecule according to claim 6 into at least one member of a population of chlamydomonas reinhardtii cells and producing a transformant-containing population;

(b) cloning and retaining a cell of said transformant-containing population that displays the phenotype induced by said transforming DNA molecule.

13. A Chlamydomonas reinhardtii cell transformed with a nucleic acid molecule according to claim 1.

14. A DNA molecule comprising the EcoR I-Sal I OEE1-derived endonuclease restriction fragment of plasmid pSB101.

15. A nucleic acid molecule suitable for transforming Chlamydomonas reinhardtii comprising a promoter operatively linked to a structural gene heterologous to said promoter, said promoter comprising the EcoR I-Sal I OEE1-derived endonuclease restriction fragment of plasmid PSB101.

FIGURE 1

```
         .-150           .              .              .-100          .
         CCGCGCGCGCAAGCACCGATCGGGCTGTACGGCTAGCGAGATCAATTTCCGTACCGCGTCGGGGCAGGCATGAA

              .              .-50           .              .
         GCGCGTTGTTTCTCTTACGGAGTTGTCCCCCGTCAACCGCTAGTCGACCATTTCTCCTTTGCACACCACAGCAGCAAAG

         ATG GCC CTC CGC GCT GCC CAG TCC GCT AAG GCC GGT GTC CGT GCC GCC CGG CCC AAC CGT
    1.    M   A   L   R   A   A   Q   S   A   K   A   G   V   R   A   A   R   P   N   R

         GCC ACC GCC GTG GTG TGC AAG GCG CAG AAG GTC GGC CAG GCC GCC GCT GCC GCT GCT CTG
   21.    A   T   A   V   V   C   K   A   Q   K   V   G   Q   A   A   A   A   A   A   L

         GCC ACC GCC ATG GTT GCC GGG TCG GCC AAC GCC CTG ACC TTC GAC GAG ATT CAG GGC CTG
   41.    A   T   A   M   V   A   G   S   A   N   A   L   T   F   D   E   I   Q   G   L

         ACC TAC CTG CAG GTC AAG GGC TCC GGC ATT GCC AAC ACC TGC CCC GTG CTG GAG AGC GGC
   61.    T   Y   L   Q   V   K   G   S   G   I   A   N   T   C   P   V   L   E   S   G

         ACC ACC AAC CTG AAG GAG CTG AAG GCC GGC TCC TAC AAG CTG GAG AAC TTC TGC ATT GAG
   81.    T   T   N   L   K   E   L   K   A   G   S   Y   K   L   E   N   F   C   I   E

         CCC ACC TCT TTC ACC GTG AAG GAG GAG AGC CAG TTC AAG GGT GGC GAG ACT GAG TTT GTC
  101.    P   T   S   F   T   V   K   E   E   S   Q   F   K   G   G   E   T   E   F   V

         AAG ACC AAG CTC ATG ACC CGC CTG ACC TAC ACC CTG GAC GCC ATG TCC GGC TCG TTC AAG
  121.    K   T   K   L   M   T   R   L   T   Y   T   L   D   A   M   S   G   S   F   K

         GTC GGC TCT GAC GGC TCC GCT GAG CTG AAG GAG GAT GAC GGC ATT GAC TAC GCC GCC ACC
  141.    V   G   S   D   G   S   A   E   L   K   E   D   D   G   I   D   Y   A   A   T

         ACC GTC CAG CTG CCC GGT GGC GAG CGC GTG GCT TTC CTG TTC ACC ATC AAG CAG TTC GAT
  151.    T   V   Q   L   P   G   G   E   R   V   A   F   L   F   T   I   K   Q   F   D

         GGC AAG GGC ACC CTG GAC GGC ATC AAG GGT GAC TTC CTG GTG CCC TCG TAC CGC GGC TCG
  181.    G   K   G   T   L   D   G   I   K   G   D   F   L   V   P   S   Y   R   G   S

         TCC TTC CTG GAC CCC AAG GGC CGC GGT GGC TCG ACC GGC TAC GAC AAC GCC GTG GCT CTG
  201.    S   F   L   D   P   K   G   R   G   G   S   T   G   Y   D   N   A   V   A   L

         CCC GCC CGC GCT GAT GCC GAG GAG CTG CTG AAG GAG AAC GTG AAG ATC ACC AAG GCC CTG
  221.    P   A   R   A   D   A   E   E   L   L   K   E   N   V   K   I   T   K   A   L

         AAG GGC TCT GCC GTG TTC TCC GTT GCC AAG GTG GAC CCC GTG ACC GGC GAG ATC GCT GGT
  241.    K   G   S   A   V   F   S   V   A   K   V   D   P   V   T   G   E   I   A   G

         GTG TTC GAG TCC ATC CAG CCC TCG GAC ACT GAC CTG GGC GCC AAG CCC CCC AAG GAC ATC
  261.    V   F   E   S   I   Q   P   S   D   T   D   L   G   A   K   P   P   K   D   I

         AAG GTC ACC GGC CTG TGG TAC GCC CAG CTG AAG TAA GCGCTTTCGCCCCCCGAAGATAAACACTTCT
  291.    K   V   T   G   L   W   Y   A   Q   L   K   •

                                              .950
         GGAGGAGGAGGACGCTACTGCTTAGCATGGTCGACCGCTGCGAGGAGGAGACGTGAGCCAGGATGCTGGAGGAGCTCTA

              .1000           .              .              .1050           .
         GCGGGTGTACATCGTAGGTGCTGCTTCCGGGATTGGAAGGGTAGGCAAAGCAGTGCTGCGGGCGGGCAGACGGCGTGTC

              .              .              .1100           .              .
         AAAACGCTCGCCTGTGTGCGGTGCAGTGCTGGAGCGTTTTTGTTGAGGACGCTCAAACTCGGGTGGATTCAGAGGTCGG

              .1150           .              .              .1200           .
         GGCGCGGAGGTTGCGTGGTTGATCGAGCGGGGGCCAAACGTTGCCGCTGCCGCACGCCTGCCGTATGCGCGCCCGGCAA

              .              .1250           .              .              .1300
         CAGCCGTGGTGCTGTCGGAGCGCGAGTGCCGGTTTTGGAGGTCTAGGAGTGCTAGGTTGGTGCGAGCGCCGTGTCATCC

              .              .              .1350           .              .
         GGCTTTTCGAGAAGAAAGATACAGTGTGCCCAAAAGTGTGTGATTATTGTGTTTCATAGTGGCTCTCGAAACAAGGCCG

              .              .1400           .              .              .1450
         CTTTCCCAGTGTAGCCTGACACTTCGGAGCCCTATTTCCCCGACTCGCTTCTCGTCGTGCATGCGCTTTGCTGGCAGTA

              .              .              .1500           .              .
         GTGCAGCACCGCTGCTCTCAGTCAATAACAGGCTGGCAAAGGCCCTGTGCACAGATTAGTCATGAGGCGTGTTTGCGGC

              .              .1550           .              .
         GGCGTGTAACACCCAAGATGAGAAAAAAAAAAAAAAAAAAAAAAAAAAAAAA
```

# FIGURE 2

FIGURE 3

A

B

FIGURE 4

FIGURE 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | PROC. NATL. ACAD. SCI. USA, vol. 84, February 1987, pages 749-753; S.P. MAYFIELD et al.: "Expression of the nuclear gene coding oxygen-evolving enhancer protein 2 is required for high levels of photosynthetic oxygen evolution in Chlamydomonas reinhardtii" * Whole article * | 1-15 | C 12 N 15/00 C 12 N 1/12 |
| D,Y | CHEMICAL ABSTRACTS, vol. 104, no. 5, 3rd February 1986, page 315, abstract no. 31582m, Columbus, Ohio, US; S.E. HASNAIN et al.: "DNA-mediated transformation of Chlamydomonas reinhardi cells: use of aminoglycoside 3'-phosphotransferase as a selectable marker", & MOL. CELL. BIOL. 1985, 5(12), 3647-50 * Abstract * | 1-15 | |
| D,Y | CHEMICAL ABSTRACTS, vol. 97, no. 1, 5th July 1982, page 153, abstract no. 1431f, Columbus, Ohio, US; J.D. ROCHAIX et al.: "Transformation of the green alga Chlamydomonas reinhardii with yeast DNA", & NATURE (LONDON) 1982, 296(5852), 70-2 * Abstract * | 1-15 | **TECHNICAL FIELDS SEARCHED (Int. Cl.4)** C 12 N |

-/-

The present search report has been drawn up for all claims

| Place of search | Date of completion of the search | Examiner |
|---|---|---|
| THE HAGUE | 04-09-1989 | PULAZZINI A.F.R. |

European Patent Office

**EUROPEAN SEARCH REPORT**

Application Number

EP 89 30 4520

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | CHEMICAL ABSTRACTS, vol. 101, no. 3, 16th July 1984, pages 177-178, abstract no. 18462r, Columbus, Ohio, US; J.D. ROCHAIX et al.: "Construction and characterization of autonomously replicating plasmids in the green unicellular alga Clamydomonas reinhardii", & CELL (CAMBRIDGE, MASS.) 1984, 36(4), 925-31 <br> * Abstract * <br> --- | | |
| Y | CHEMICAL ABSTRACTS, vol. 102, no. 25, 24th June 1985, page 180, abstract no. 216244e, Columbus, Ohio, US; J.D. ROCHAIX: "Transformation in the green alga Chlamydomonas reinhardii", & GENET. ENG. (N. Y.) 1984, 6, 17-30 <br> * ABstract * <br> --- | 1-15 | |
| D,Y | THE EMBO JOURNAL, vol. 6, no. 2, 1987, pages 313-318, IRL Press Ltd, Oxford, GB; S.P. MAYFIELD et al.: "Expression of the nuclear encoded OEE1 protein is required for oxygen evolution and stability of photosystem II particles in Clamydomonas reinhardtii" <br> --- | 1-15 | TECHNICAL FIELDS SEARCHED (Int. Cl.4) |
| O,P X | JOURNAL OF CELLULAR BIOCHEMISTRY, UCLA SYMPOSIUM, MOLECULAR PLANT BIOLOGY, 27th March - 7th April 1989, page 229, Alan R. Liss, Inc., New York, US; S.P. MAYFIELD et al.: "Stable nuclear transformation of Chlamydomonas reinhardtii using a C. reinhardtii gene as the selectable maker" <br> * Whole article * <br> --- -/- | 1-15 | |

The present search report has been drawn up for all claims

| Place of search | Date of completion of the search | Examiner |
|---|---|---|
| THE HAGUE | 04-09-1989 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)

# EUROPEAN SEARCH REPORT

European Patent Office

Application Number

EP 89 30 4520

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | ABSTRACTS OF THE ANNUAL MEETING OF THE AMERICAN SOCIETY FOR MICROBIOLOGY, vol. 86, no. 0, 1986, page 142, abstract no. H-89; G. STIEVENART et al.: "Transformation of Chlamydomonas reinhardtii CW15 with a plasmid containing C. reinhardtii" * Abstract * ----- | 1 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 04-09-1989 | PULAZZINI A.F.R. |